# EUROPÄISCHE PATENTANMELDUNG

(11) **EP 3 788 996 A1**
(43) Veröffentlichungstag der Anmeldung: **10.03.2021**
(21) Anmeldenummer: 19196285.1
(22) Anmeldetag: 09.09.2019
(51) Int. Cl.: A61F 13/00, A61B 5/00, A61F 13/02

(54) **VERBANDSMATERIAL**

(71) Anmelder: Aktimed GmbH, 69117 Heidelberg (DE)
(72) Erfinder: Kroker-Hohmann, Sabine, 69120 Heidelberg (DE)
(74) Vertreter: Maiwald Patent- und Rechtsanwaltsgesellschaft mbH

(57) **Zusammenfassung**

Die vorliegende Erfindung betrifft ein Verbandsmaterial zum Tragen auf der Haut eines Benutzers. Das Verbandsmaterial umfasst ein Trägermaterial, mindestens einen physiologischen Sensor, der mit dem Trägermaterial verbunden ist, wobei der Sensor zum Erfassen von mindestens einem Wert eines Stoffwechselprodukts des Benutzers oder zum Erfassen von mindestens einem Wert eines Mineralstoffes des Benutzers ausgebildet ist, und eine Schnittstelle, die zum Ausgeben des erfassten Werts ausgebildet ist.

## Beschreibung

Die vorliegende Erfindung betrifft ein Verbandsmaterial zum Tragen auf der Haut eines Benutzers.

Viele Verletzungen des Bewegungsapparates, wie z.B. Verletzungen von Muskeln, Bänder und Sehnen, werden durch teilweise oder vollständige Ruhigstellung der jeweiligen, die Verletzung aufweisenden Körperpartien behandelt. Die vollständige Ruhigstellung erfolgt beispielsweise durch Gipsverbände. Der Nachteil bei einer vollständigen Ruhigstellung ist, dass Störungen der Durchblutung auftreten können oder beispielsweise Gelenke nach einem längeren Zeitraum der Ruhigstellung in ihrer Bewegungsfreiheit eingeschränkt sind, die nur durch zeitaufwendige Physiomaßnahmen wiederhergestellt werden kann. Im Gegensatz dazu erlauben Tapeverbände eine teilweise Ruhigstellung, durch die einige der mit einer vollständigen Ruhigstellung einhergehenden Nachteile vermieden werden können. Ein Tapeverband stützt und entlastet zugleich und ermöglicht eine funktionelle Belastung in begrenztem Maße.

Aus EP 1 924 296 B1 ist ein Verbandsmaterial bekannt, das mit einem natürlichen Wirkstoff, wie z.B. Arnica montana, Rhus toxicodendron oder Ruta graveolens, versehen ist, mittels dessen nicht nur eine teilweise Ruhigstellung einer verletzten Körperpartie ermöglicht wird, sondern zudem durch den darin vorgesehenen Wirkstoff auch die Regeneration und der Heilungsprozess beschleunigt wird.

Aus CN 108579063 A ist ein Tapeverband mit darin integriertem Bewegungssensor bekannt.

KR 101843162 B1 offenbart ein kinesiologisches Tape, das über eine Sensorik verfügt, mit Hilfe derer die Aktivität oberflächennaher Muskelfasern (Elektromyogramm) erfasst werden kann.

Eine Aufgabe der vorliegenden Erfindung besteht darin, ein Verbandsmaterial zur Verfügung zu stellen, das neben der mechanischen Wirkungsweise dem Benutzer einen zusätzlichen Mehrwert bei der Prophylaxe und der Behandlung von physiologischen Beschwerden bietet. Diese Aufgabe wird durch den Gegenstand des Patentanspruchs 1 gelöst. Optionale bzw. bevorzugte Merkmale der Erfindung sind in den abhängigen Patentansprüchen 2 bis 16 angegeben.

Gemäß der Erfindung wird ein Verbandsmaterial zum Tragen auf der Haut eines Benutzers zur Verfügung gestellt. Erfindungsgemäß umfasst das Verbandsmaterial ein Trägermaterial, mindestens einen physiologischen Sensor, der mit dem Trägermaterial verbunden ist, wobei der physiologische Sensor zum Erfassen von mindestens einem Wert eines Stoffwechselprodukts des Benutzers oder zum Erfassen von mindestens einem Wert eines Mineralstoffes des Benutzers ausgebildet ist, und eine Schnittstelle, die zum Ausgeben des erfassten Werts ausgebildet ist.

Vorzugsweise ist der physiologische Sensor zum Erfassen von mindestens einem Wert eines auf der Haut des Benutzers auftretenden Stoffwechselprodukts ausgebildet.

Noch bevorzugter ist der physiologische Sensor zum Erfassen mehrerer Werte von auf der Haut des Benutzers auftretenden Stoffwechselprodukten ausgebildet.

Vorteilhaft ist es, wenn der physiologische Sensor zum Erfassen von Laktat, Glukose, Kalium- und Natriumionen ausgebildet ist.

Besonders bevorzugt ist der physiologische Sensor zusätzlich zum Erfassen der Hauttemperatur des Benutzers ausgebildet ist.

Gemäß einer bevorzugten Ausführungsform der Erfindung ist der physiologische Sensor ein Schweißsensor oder ein Hautleitfähigkeitssensor.

Bevorzugt ist das Trägermaterial entlang seiner Längsachse um bis zu 95% dehnbar.

Noch bevorzugter ist das Trägermaterial entlang seiner Querachse um höchstens 5% dehnbar.

Besonders bevorzugt ist der physiologische Sensor mit dem Trägermaterial unter Verwendung eines biokompatiblen Haftmittels verbunden, vorzugsweise verklebt.

Ganz besonders bevorzugt weist das Trägermaterial ein erstes Trägermaterial, das beim bestimmungsgemäßen Gebrauch des Verbandsmaterials mit der Haut in Kontakt tritt, und ein zweites Trägermaterial auf.

Vorteilhaft ist es, wenn das erste Trägermaterial Zinkoxid, Kautschuk und Acryl umfasst.

Ebenso ist es vorteilhaft, wenn Zinkoxid in 1% bis 10% (w/w), Kautschuk in 1% bis 10% (w/w) und Acryl in 80% bis 98% (w/w) vorliegt.

Besonders vorteilhaft ist es, wenn Zinkoxid in 5% (w/w), Kautschuk in 5% (w/w) und Acryl in 90% (w/w) vorliegt.

Gemäß einer weiteren bevorzugten Ausführungsform der Erfindung umfasst das zweite Trägermaterial Baumwolle.

Weiterhin bevorzugt weist das Trägermaterial ein drittes Trägermaterial auf, das zur Abgabe von einem pharmazeutisch aktiven Wirkstoff oder von einem pflanzlichen Extrakt oder von einem Homöopathika ausgebildet ist.

Vorzugsweise ist das Verbandsmaterial ein kinesiologisches Tape, ein Zinkleimverband oder ein Pflasterverband.

Die Erfindung wird nun rein beispielhaft anhand einer bevorzugten Ausführungsform der Erfindung beschrieben.

Wenngleich in der vorliegenden Beschreibung von Verbandsmaterial die Rede ist, so kann hierunter jedwedes Pflaster, wie z.B. ein Gitterpflaster, Tape, wie z.B. ein kinesiologisches Tape, oder auch ein Zinkleimverband verstanden werden.

Nach einer bevorzugten Ausführungsform umfasst das erfindungsgemäße Verbandsmaterial ein Trägermaterial. Diese Trägermaterial ist vorzugsweise in Längsrichtung um bis zu 95% dehnbar, während es in Querrichtung dazu um höchsten 5% dehnbar ist, bevor das Trägermaterial bei Überschreiten dieser Dehnungsgrenzen zu reissen beginnt. Durch die unterschiedlichen Dehnbarkeiten wird durch das Verbandsmaterial die erwünschte mechanische Stabilität zu Präventionszwecken oder beim teilweisen Ruhigstellen von verletzten Körperpartien erzielt.

Das Trägermaterial umfasst vorzugsweise ein erstes Trägermaterial, das bei der bestimmungsgemäßen Benutzung mit der Haut des Benutzers in Kontakt tritt, und ein zweites Trägermaterial. Das erste Trägermaterial umfasst vorzugsweise Zinkoxid in 1% bis 10% (w/w), Kautschuk in 1% bis 10% (w/w) und Acryl in 80% bis 98% (w/w), noch bevorzugter Zinkoxid in 5% (w/w), Kautschuk in 5% (w/w) und Acryl in 90% (w/w). Das zweite Trägermaterial umfasst bevorzugt Baumwolle.

Gemäß einer weiteren bevorzugten Ausführungsform umfasst das Trägermaterial ein drittes Trägermaterial, das zur vorzugsweise kontrollierten Abgabe von mindenstens einem pharmazeutisch aktiven Wirkstoff, wie z.B. CB1 und CB2 Arzneistoffe und/oder Ibuprofen, Diclophenac ausgebildet ist. Des Weiteren können auch pflanzliche oder Homöopathika, wie z.B. Arnica Montana D6 abgegeben werden.

Verbunden mit dem Trägermaterial ist mindestens ein physiologischer Sensor, der zum Erfassen, vorzugsweise Messen von mindestens einem Wert eines Stoffwechselprodukts des Benutzers oder zum Erfassen, vorzugsweise Messen eines Werts eines Mineralstoffes des Benutzers ausgebildet ist. Denkbar in diesem Zusammenhang sind physiologische Sensoren, wie zum Beispiel Schweißsensoren oder Hautleitfähigkeitssensoren. Der physiologische Sensor ist dabei vorzugsweise mit dem ersten Trägermaterial verklebt, und zwar unter Verwendung eines biokompatiblen Haftmittels, beispielsweise eines biokompatiblen Klebers.

Menschlicher Schweiß enthält eine Reihe von Informationen, die in physiologischer Hinsicht von Bedeutung sind, was den menschlichen Schweiß gerade für nicht-invasive, tragbare Sensoren attraktiv macht. Im Falle eines Schweißsensors werden insbesondere die auf der Haut des Benutzers auftretenden Stoffwechselprodukte, wie Laktat, Glukose sowie Kalium- und Natriumionen erfasst. Da die Konzentration dieser Messwerte temperaturabhängig ist, wird vorzugsweise zusätzlich die Hauttemperatur des Benutzers von dem physiologischen Sensor erfasst, vorzugsweise gemessen.

Vorzugsweise kann das Trägermaterial auch mit einem für Blinde lesbaren Blindenschriftzeichen versehen sein, das auf die Art des physiologischen Sensors oder Verbandsmaterials hinweist.

Das Verbandsmaterial weist darüber hinaus eine Schnittstelle auf, welche die erfassten Werte, vorzugsweise Messwerte des physiologischen Sensors ausgibt, vorzugsweise kabellos ausgibt. Die kabellose Ausgabe erfolgt vorzugsweise mittels einer Bluetooth-Technologie an mobile Endgeräte. Diese mobilen Endgeräte wiederum geben unter Verwendung entsprechender Blockchain-Technologien diese Daten an externe Netzwerke weiter, so dass auch Krankenkassen sowie Krankenversicherungen wie auch Ärzte in die weitere Diagnose mit einbezogen werden können.

Der Kern der Erfindung ist die gezielte Kombination der Anwendung von Verbandsmaterialien, wie zum Beispiel kinesiologischer Tapes, Zinkleimverbänden oder Pflastern, mit der Erfassung und Überwachung physiologischer Parameter durch die in den Verbandsmaterialien integrierten Sensoren. Abhängig von den erfassten physiologischen Parametern kann eine weitergehende lokale, z.B. durch in dem Verbandsmaterial enthaltene Wirkstoffe, die auch pharmazeutisch aktiv sein können, oder orale Verabreichung wirksamer unterstützender Substanzen erfolgen.

Das erfindungsgemäße Verbandsmaterial ist in vielerlei Hinsicht vorteilhaft.

Sportler beispielsweise kleben sich Tapes oder Verbände als mechanische Unterstützung zur Steigerung ihrer Leistungsfähigkeit oder indikationsbezogen an entsprechende Körperpartien auf. Indikationen hierbei können präventiver Natur sein oder betreffen Verletzungen, wie z.B. einen Muskelfaserriss, einen Tennisellenbogen, Kniegelenksbeschwerden, Achillessehnenbeschwerden, Rückenbeschwerden usw.. Durch die erfindungsgemäße integrierte Sensorik in dem Verbandsmaterial haben Sportler nun zusätzlich zur mechanischen Unterstützung noch die Möglichkeit festzustellen, welche Art von Mineralstoffen, Vitaminen oder andere Substanzen nicht in ausreichendem Maße im Körper vorhanden sind, wie dies durch die Erfassung der in dem menschlichen Schweiß enthaltenen physiologischen Substanzen feststellbar ist. Durch die Bestimmung möglicher Defizite und die Weiterleitung der entsprechenden Informationen an externe Netzwerke können Empfehlungen sodann ausgesprochen werden, wie der Sportler wieder leichter und schneller diese Defizite ausgleichen kann.

Frauen können sich solche Verbände bei der Feststellung von bestimmten Befindlichkeiten, wie z.B. Schlafstörungen, Eisenmangel, Jodmangel etc., auf entsprechende Körperpartien aufkleben. Je nach Indikation wird das erfindungsgemäße Verbandsmaterial an unterschiedichen Körperpartien aufgeklebt. So wird bei Menstruationsbeschwerden das Verbandsmaterial im Lendenwirbelsäulenbereich und Unterbauch aufgeklebt, während es bei Schlafstörungen auf die Stirn oder entsprechende Akupunkturpunkte angebracht wird. Die von dem physiologischen Sensor gelieferten Werte hinsichtlich Magnesiumgehalt und Eisenwerte werden von dem Verbandsmaterial kabellos an ein mobiles Endgerät übertragen. Mithilfe einer entsprechenden App kann sodann eine Empfehlung ausgesprochen werden, welche Nahrungsergänzungsmittel/Medikamente zuzuführen sind, damit die Gesundung der Frau bzw. eine Linderung der Befindlichkeiten erzielt werden kann.

Bei sog. Best Agern können die erfindungsgemäßen Verbandsmaterialien zur allgemeinen Unterstützung des Wohlbefindens angewendet werden. Nachdem in Echtzeit etwaige Mineralstoffe erfasst und analysiert wurden, wird via App ein Vorschlag für die Zufuhr der fehlenden Substanzen an den Benutzer übermittelt. Nach Einnahme der Substanzen kann zum vorherigen Unwohlzustand der Erfolg des Wohlbefindens auf dem mobilen Datenträger beobachtet werden.

Die Erfindung ermöglicht auf diese Weise eine kontrollierte und gezielte Behandlung von häufig auftretenden Beschwerden. Durch den ganzheitlichen Ansatz werden die therapeutischen wie die diagnostischen Bedürfnisse des Benutzers abgedeckt. Es kann selbstständig eine an die Person und die Situation angepasste Behandlung erfolgen. Durch diese Art der Überwachung, die auch in Echtzeit erfolgen kann, kann eine unsinnige Einnahme von "Supplements" verhindert werden.

## Patentansprüche

1. Verbandsmaterial zum Tragen auf der Haut eines Benutzers, umfassend:
- ein Trägermaterial;
- mindestens einen physiologischen Sensor, der mit dem Trägermaterial verbunden ist, wobei der physiologische Sensor zum Erfassen von mindestens einem Wert eines Stoffwechselprodukts des Benutzers oder zum Erfassen von mindestens einem Wert eines Mineralstoffes des Benutzers ausgebildet ist, und
- eine Schnittstelle, die zum Ausgeben des erfassten Werts ausgebildet ist.

2. Verbandsmaterial nach Anspruch 1, wobei der physiologische Sensor zum Erfassen von mindestens einem Wert eines auf der Haut des Benutzers auftretenden Stoffwechselprodukts ausgebildet ist.

3. Verbandsmaterial nach Anspruch 1 oder 2, wobei der physiologische Sensor zum Erfassen mehrerer Werte von auf der Haut des Benutzers auftretenden Stoffwechselprodukten ausgebildet ist.

4. Verbandsmaterial nach einem der vorhergehenden Ansprüche, wobei der physiologische Sensor zum Erfassen von Laktat, Glukose, Kalium- und Natriumionen ausgebildet ist.

5. Verbandsmaterial nach einem der vorhergehenden Ansprüche, wobei der physiologische Sensor zusätzlich zum Erfassen der Hauttemperatur des Benutzers ausgebildet ist.

6. Verbandsmaterial nach einem der vorhergehenden Ansprüche, wobei der physiologische Sensor ein Schweißsensor oder ein Hautleitfähigkeitssensor ist.

7. Verbandsmaterial nach einem der vorhergehenden Ansprüche, wobei das Trägermaterial entlang seiner Längsachse um bis zu 95% dehnbar ist.

8. Verbandsmaterial nach einem der vorhergehenden Ansprüche, wobei das Trägermaterial entlang seiner Querachse um höchstens 5% dehnbar ist.

9. Verbandsmaterial nach einem der vorhergehenden Ansprüche, wobei der physiologische Sensor mit dem Trägermaterial unter Verwendung eines biokompatiblen Haftmittels verbunden, vorzugsweise verklebt ist.

10. Verbandsmaterial nach einem der vorhergehenden Ansprüche, wobei das Trägermaterial ein erstes Trägermaterial, das beim bestimmungsgemäßen Gebrauch des Verbandsmaterials mit der Haut in Kontakt tritt, und ein zweites Trägermaterial aufweist.

11. Verbandsmaterial nach Anspruch 10, wobei das erste Trägermaterial Zinkoxid, Kautschuk und Acryl umfasst.

12. Verbandsmaterial nach Anspruch 11, wobei Zinkoxid in 1% bis 10% (w/w), Kautschuk in 1% bis 10% (w/w) und Acryl in 80% bis 98% (w/w) vorliegt.

13. Verbandsmaterial nach Anspruch 11 oder 12, wobei Zinkoxid in 5% (w/w), Kautschuk in 5% (w/w) und Acryl in 90% (w/w) vorliegt.

14. Verbandsmaterial nach einem der Ansprüche 10 bis 13, wobei das zweite Trägermaterial Baumwolle umfasst.

15. Verbandsmaterial nach einem der vorhergehenden Ansprüche, wobei das Trägermaterial ein drittes Trägermaterial aufweist, das zur Abgabe von einem pharmazeutisch aktiven Wirkstoff oder von einem pflanzlichen Extrakt oder von einem Homöopathika ausgebildet ist.

16. Verbandsmaterial nach einem der vorhergehenden Ansprüche, wobei das Verbandsmaterial ein kinesiologisches Tape, ein Zinkleimverband oder ein Pflasterverband ist.
